(19) **Europäisches Patentamt European Patent Office Office européen des brevets**

(11) **EP 4 518 849 B1**

(12) **EUROPEAN PATENT SPECIFICATION**

(45) Date of publication and mention of the grant of the patent:
**08.04.2026 Bulletin 2026/15**

(21) Application number: **23727415.4**

(22) Date of filing: **05.05.2023**

(51) International Patent Classification (IPC):
*A61K 31/235* (2006.01)   *A61K 31/4184* (2006.01)
*A61K 9/00* (2006.01)   *A61P 17/00* (2006.01)
*A61P 17/04* (2006.01)   *A61P 17/06* (2006.01)
*A61P 17/08* (2006.01)   *A61P 17/10* (2006.01)
*A61P 17/16* (2006.01)   *A61P 17/18* (2006.01)
*A61K 8/37* (2006.01)   *A61K 8/41* (2006.01)
*A61K 8/49* (2006.01)   *A61K 47/10* (2017.01)
*A61K 47/12* (2006.01)   *A61K 47/14* (2017.01)
*A61K 47/18* (2017.01)   *A61K 47/36* (2006.01)
*A61Q 17/04* (2006.01)

(52) Cooperative Patent Classification (CPC):
(C-Sets available)
A61K 31/235; A61K 8/37; A61K 8/415;
A61K 8/4946; A61K 9/0014; A61K 31/4184;
A61K 47/10; A61K 47/12; A61K 47/14;
A61K 47/183; A61K 47/36; A61P 17/00;
A61P 17/04; A61P 17/06; A61P 17/08;   (Cont.)

(86) International application number:
**PCT/IB2023/054703**

(87) International publication number:
**WO 2023/214374 (09.11.2023 Gazette 2023/45)**

(54) **COMPOSITION FOR TOPICAL APPLICATION FOR THE PHOTOTHERAPEUTIC TREATMENT OF INFLAMMATORY-BASED SKIN DISEASES**

ZUSAMMENSETZUNG ZUR TOPISCHEN ANWENDUNG ZUR PHOTOTHERAPEUTISCHEN BEHANDLUNG VON ENTZÜNDLICHEN HAUTERKRANKUNGEN

COMPOSITION POUR APPLICATION TOPIQUE POUR LE TRAITEMENT PHOTOTHÉRAPEUTIQUE DE MALADIES CUTANÉES INFLAMMATOIRES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.05.2022 IT 202200009212**

(43) Date of publication of application:
**12.03.2025 Bulletin 2025/11**

(73) Proprietor: **Giuliani S.p.A.**
**20129 Milano (IT)**

(72) Inventors:
• **GIULIANI, Giammaria**
 **6926 Montagnola (CH)**
• **RINALDI, Fabio**
 **20122 Milano (IT)**
• **PINTO, Daniela**
 **20151 Milano (IT)**
• **MASCOLO, Antonio**
 **20126 Milano (IT)**
• **MARZANI, Barbara**
 **27020 Carbonara Al Ticino (IT)**

(74) Representative: **Cattaneo, Elisabetta et al**
**Notarbartolo & Gervasi S.p.A.**
**Viale Achille Papa, 30**
**20149 Milano (IT)**

(56) References cited:
EP-A1- 2 939 710          WO-A2-2010/076731
US-A1- 2014 121 732      US-A1- 2014 323 950
US-B2- 9 968 800

(52) Cooperative Patent Classification (CPC): (Cont.)
A61P 17/10; A61P 17/16; A61P 17/18; A61Q 17/04

C-Sets
A61K 31/235, A61K 2300/00;
A61K 31/4184, A61K 2300/00

**Description**

FIELD OF THE INVENTION

The invention concerns a composition for topical application for the phototherapeutic treatment of inflammatory skin diseases.

The present invention originates in the pharmaceutical sector, specifically in the field of products for dermatological use.

In particular, the present invention concerns compositions for cutaneous application containing a combination of compounds which shield some ultraviolet UV radiations while allowing ultraviolet radiation in a selected wavelength range, suitable for achieving the phototherapeutic treatment of dermatological conditions with an inflammatory component, to pass through.

STATE OF THE ART

Ultraviolet radiation is an electromagnetic radiation originating from the sun and has a wavelength immediately lower than visible light and higher than that of X-rays. The intensity of the electromagnetic radiation is expressed with the UV index which is divided into bands defined differently according to the fields of study.

In the life sciences sector the UV wavelength range is typically divided into three bands: UV-A (400-315 nm), UV-B (315-280 nm) and UV-C (280-100 nm).

Although the sun emits photons in a wide range of frequencies, including those of ultraviolet light in all three UV-A, UV-B and UV-C bands, in the medical field the studies on the effects of ultraviolet rays on human health focus mainly on UV-A and UV-B, since almost 100% of UV-C does not reach the earth's surface, being filtered by the atmospheric ozone layer.

In the medical-dermatological field there are numerous studies on the effects of skin exposure to UV radiation. The interest in this topic is motivated by the fact that ultraviolet radiation represents one of the environmental factors most responsible for skin aging and the development of skin diseases, which can be of a benign nature and transient duration, such as burns and rashes solar.

Exposure to sun rays also has a certain relevance in the immunological field, since prolonged exposure can weaken the local immune response making the skin more prone to develop inflammatory dermatological diseases.

Exposure to different frequencies of ultraviolet (UV) light can cause different kinds of damage to the skin. UVB rays mainly cause burning and redness. UVB rays stimulate the production of melanin, the pigment that acts as a natural protection factor and gives the skin a darker color than usual. UVA rays penetrate deeply into the skin layers causing a deterioration of the collagen and elastin fibers and the collapse of the skin support, and accelerate the aging process by increasing the formation of wrinkles.

Furthermore, the intense exposure of the skin to UV rays can cause even deep tissue damage and trigger an inflammatory process which manifests itself with typical local manifestations such as redness, itching, skin rashes, pain, and skin thickening.

It has been observed that there are multiple damages resulting from sun exposure and they can affect both the DNA of keratinocytes and the protein component of dermis and epidermis.

Scientific studies have shown that UVB rays mainly interact directly with the DNA of skin cells by modifying it, while UVA rays indirectly interact with the skin through the formation of radical species that accelerate cellular oxidative processes.

In this general framework, solar products play the role of skin protective agents against exposure to sunlight, while ultraviolet rays represent an external agent potentially harmful to skin health.

To limit the risks related to excessive exposure to sunlight, the application of solar products is used. These products are topical preparations containing compounds that block a broad spectrum of electromagnetic radiation in the UV spectrum. The main purpose of sunscreens or solar products is to intercept solar radiation and prevent it from interacting with the skin, protecting the latter from damage and exposure effects.

The shielding effect of solar products can be almost total or partial depending on the composition, the amounts of shielding agents present in the formulation, and the quantity applied to the skin.

The shielding agents present in solar products, and which have the ability to interact with UV radiation, are also known as sun filters. These filters are divided into categories such as UVA filters, UVB filters and combined filters which partially overlap the protection in the UVA, UVB spectrums, depending on the ability to interact with ultraviolet radiation of a given wavelength. Sunscreens contain specific ingredients that can interact with the incident radiation through three fundamental mechanisms: reflection, diffusion (scattering) and absorption.

The level of protection is mainly determined by two characteristics: the filtering capacity of the compound and the amount applied to the skin.

Low concentrations of shielding agents correspond to solar products with a lower protection index, while high concentrations correspond to products with high protection across the entire UV spectrum.

US 9 968 800 B2 describes a method of delivering a UV phototherapy to a patient to treat a skin disorder (psoriasis) in the 300-320 nm range by applying a UV transparent dressing which may comprise a composition that blocks UVA light and let UVB radiation pass through. US 2014/323950 A1 describes a composition comprising a skin-lightening agent for use in the phototherapeutic and cosmetic treatment of psoriasis, atopic dermatitis, acne, eczema, inflammation and reddening of the skin. WO 2010/076731 A2 describes a sunscreen composition containing one or more active UV radiation absorbers, which however permit the UVB radiation to enter the skin in an amount sufficient for the body to produce a healthful and disease-opposing quantity of vitamin D3 (wavelength 295-315 nm).

The present invention originates from the need to exploit in the dermatological field the therapeutic effects associated with a low exposure of the skin to ultra-violet rays, while maintaining adequate sun protection.

Within the scope of the invention, a technical problem is represented by the need to combine in a single product the filtering or shielding properties typical of a sunscreen product with the curative properties of ultraviolet radiation on some dermatological diseases.

One of the objects of the present invention therefore consists in providing a product for topical application for the phototherapeutic treatment of skin diseases which respond to exposure to ultraviolet rays of selected wavelengths.

Another object consists in providing a composition for topical use to carry out the phototherapeutic treatment of skin diseases, in particular with an inflammatory component, substantially reducing or eliminating the side effects connected with the exposure of the skin to ultraviolet radiation.

## SUMMARY OF THE INVENTION

In accordance with a general aspect of the present invention, the inventors have found that by combining selected compounds, a composition for topical use is obtained, which protects the skin from the action of ultraviolet rays, while allowing UVB radiations with a wavelength in a range selected to allow treatment of skin diseases with an inflammatory component to pass through.

Advantageously, the combination composition for topical use performs the dual effect of protecting the skin from the harmful effects caused by exposure to a broad ultraviolet radiation band and of phototherapeutic treatment of skin diseases which benefit from exposure to ultraviolet radiation of selected wavelength.

According to the invention, said skin disease with an inflammatory component is selected from the group consisting of psoriasis, atopic dermatitis, acne, acne inversa/hidradenitis suppurativa, rosacea, seborrheic dermatitis, eczematous dermatitis, vitiligo, folliculitis, pityriasis rosea, chronic lichenoid pityriasis, pityriasis lichenoides et varioliformis acuta, chronic urticaria, eosinophilic pustular folliculitis, polymorphous light eruption, pruritus and prurigo nodularis.

This effect is achieved by combining specific compounds which shield some ultraviolet radiation, in particular UVA, and allowing a gap in the ultraviolet UVB radiation in the range from 295 to 320 nm to pass through.

Therefore, the object of the present invention is a composition for topical application for use in the phototherapeutic treatment of a skin disease with an inflammatory component, wherein said composition comprises a combination of compounds which shield ultraviolet radiations and allow ultraviolet radiation with a wavelength in the range from 295 to 320 nm to pass through at least partially, and a pharmaceutically acceptable carrier, and wherein said combination comprises the compounds diethylhexyl 2,6-naphthalate, diethylamino hydroxybenzoyl hexyl benzoate, phenyl dibenzimidazole tetrasulfonate, and pharmaceutically acceptable salts thereof.

The combination composition described herein shields ultraviolet radiation in a broad spectrum while allowing a cut-off passage in the UVB region.

In the cut-off region selected solar radiations are allowed to pass through in order to stimulate specific biological responses, triggered by the interaction of the skin with the incident radiations.

According to preferred embodiments, the combination composition described herein allows ultraviolet radiation with a wavelength from 300 to 315 nm to pass through.

In accordance with certain aspects of the invention, the combination composition for use according to the invention finds application preferably in the treatment of skin diseases with an inflammatory component such as psoriasis, atopic dermatitis, vitiligo, acne, and rosacea.

According to certain aspects, the composition for use described herein finds application in the phototherapeutic treatment when the ultraviolet radiation originates from artificial lamps which have emission spectra falling within the UVB wavelengths.

In accordance with a second aspect, the present invention provides the cosmetic non-therapeutic use of a composition for topical application to improve the aesthetic appearance of skin affected by skin redness, skin flushing, wherein said composition comprises a combination of the compounds diethylhexyl 2,6-naphthalate, diethylamino hydroxybenzoyl hexyl benzoate, phenyl dibenzimidazole tetrasulfonate, and pharmaceutically acceptable salts thereof, and a cosmetically acceptable carrier for shielding ultraviolet radiation and allowing ultraviolet radiation with a wavelength in the range from 295 to 320 nm, in particular from 300 to 315nm, to pass through, at least partially.

Some aspects and advantages of the invention are further described with reference to the attached figures.

BRIEF DESCRIPTION OF THE DRAWINGS

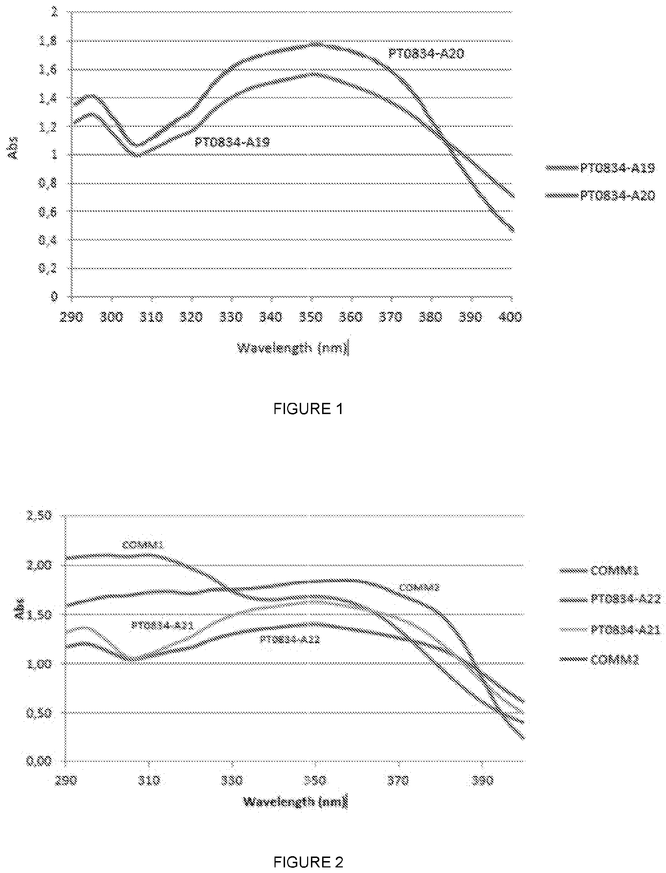

Figure 1 shows two graphs in which the abscissa reports the wavelength values of the electromagnetic radiation, and the ordinate reports the absorbance values of two sample compositions for use according to the invention, identified with the acronyms PT0834-A19 and PT0834-A20. Both graphs show an absorbance valley in the wavelength region of 295-320 nm. The UV rays absorption decrease is particularly evident in the wavelength range from 305 to 315 nm. Figure 2 illustrates four graphs showing the absorbance values recorded by exposing four samples to UV at different wavelengths. Of the four samples tested, the compositions PT0834-A21 and PT0834-A22 had a formulation according to the invention and showed a marked reduction in absorption in the ultraviolet UVB region from 295 to 320 nm. The two commercially available compositions COMM 1 and COMM 2 with protection index SPF15 showed a substantially constant absorption of UVB radiation in the region of interest from 295 to 320 nm. These two formulations were found to be unsuitable for use in phototherapeutic treatment according to the invention.

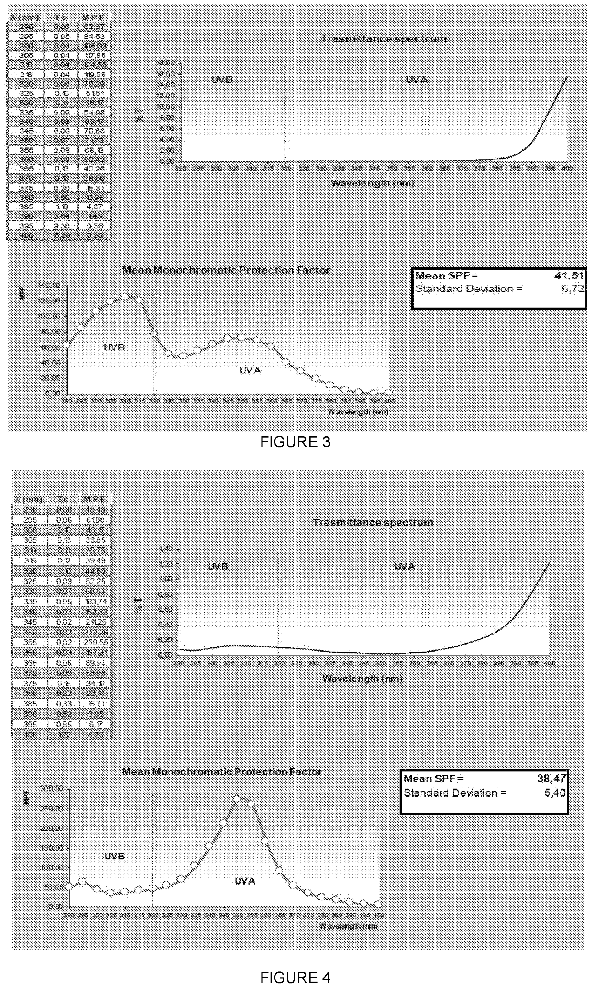

Figure 3 shows two graphs, one relating to transmittance and the other to absorbance spectrum (Mean Monochromatic Protection Factor - Mean SPF) of a commercially available solar product, identified with the acronym COMM3, having the qualitative composition described in Example 5 (COMM3). The absorbance graph of COMM3 composition with SPF 50 protection index shows an increasing trend of UVB radiation in the region of interest from 295 to 320 nm, an opposite effect to that required for uses in phototherapy according to the invention.

Figure 4 shows two graphs, one relating to transmittance and the other to absorbance spectrum (Mean Monochromatic Protection Factor - Mean SPF) of a composition according to the invention, identified with the acronym PT0834T20-1, having the quali-quantitative composition described in Example 5. The absorbance graph of the composition PT0834T20-1 shows a reduction or window in the absorption of UVB radiation in the region from 295 to 320 nm, more marked in the range from 305 to 315 nm, highlighting the achievement of the required effect for the uses in phototherapy according to the invention.

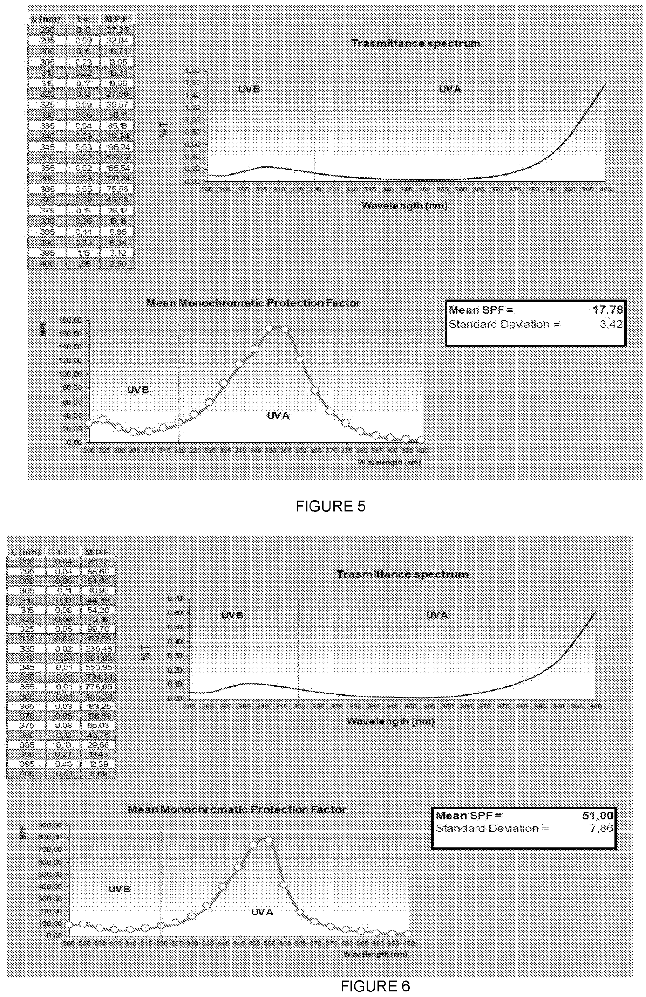

Figure 5 shows two graphs, one relating to transmittance and the other to absorbance spectrum (Mean Monochromatic Protection Factor - Mean SPF) of a composition according to the invention, identified with the acronym PT0834T20-2, having the quali-quantitative composition described in Example 5. The absorbance graph of the composition PT0834T20-2 shows a reduction or window in the absorption of UVB radiation in the region from 295 to 320 nm, more marked in the range from 305 to 310 nm, highlighting the achievement of the required effect for the uses in phototherapy according to the invention.

Figure 6 shows two graphs, one relating to transmittance and the other to absorbance spectrum (Mean Monochromatic Protection Factor - Mean SPF) of a composition according to the invention, identified with the acronym PT0834T20-3, having the quali-quantitative composition described in Example 5. The absorbance graph of the composition PT0834T20-3 shows a reduction or window in the absorption of UVB radiation in the region from 295 to 320 nm, more marked in the range from 305 to 310 nm, highlighting the achievement of the required effect for the uses in phototherapy according to the invention.

DETAILED DESCRIPTION OF THE INVENTION

The composition for the uses of the invention combines the UV rays shielding and absorption effect typical of solar products with a skin phototherapeutic effect attributable to the exposure of the skin to ultraviolet radiation in a narrow and selected wavelength range. This second effect is made possible by combining two compounds which absorb UVA radiation with a compound which has low protection against UVB rays and allows electromagnetic radiation with a wavelength from 295 to 320nm to pass through, thus stimulating specific biological reactions triggered by the interaction of the skin with UV rays of a selected wavelength. These biological responses result in a reduction of local inflammation and the treatment of skin diseases with an inflammatory component.

According to some aspects, the invention originates from the selection and combination of compounds which offer broad spectrum protection from ultraviolet radiation by allowing electromagnetic radiation with wavelengths in the range from 295 to 320 nm, selected to achieve the phototherapeutic treatment of skin diseases characterized by an inflammatory component, to pass through.

The applicant has therefore found that by combining two filtering compounds which absorb in the UVA region with a photostabilizing compound which reduces the photodegradation of Avobenzone, a composition is obtained which protects the skin from ultraviolet rays and allows radiation in the range from 295 to 320 nm of the UVB spectrum to pass through, thus determining a specific cut-off of the shielding effect in the region suitable for achieving a therapeutic effect on skin diseases.

EP 4 518 849 B1

According to one aspect, it is therefore provided a composition for topical application for use in the phototherapeutic treatment of a skin disease with an inflammatory component, wherein said composition comprises a combination of compounds which partially protect the skin from ultraviolet radiation, while allowing UVB ultraviolet radiation having a wavelength in the range from 295 to 320 nm to pass through, wherein said combination of compounds comprises diethylhexyl 2,6-naphthalate, diethylamino hydroxybenzoyl hexyl benzoate, phenyl dibenzimidazole tetrasulfonate, and pharmaceutically acceptable salts thereof, and wherein said skin disease with an inflammatory component is selected from the group consisting of psoriasis, atopic dermatitis, acne, acne inversa/hidradenitis suppurativa, rosacea, seborrheic dermatitis, eczematous dermatitis, vitiligo, folliculitis, pityriasis rosea, chronic lichenoid pityriasis, pityriasis lichenoides et varioliformis acuta, chronic urticaria, eosinophilic pustular folliculitis, polymorphous light eruption, pruritus and prurigo nodularis.

The composition according to the invention comprises a combination of compounds which allow UVB radiation with a wavelength in the range from 305 to 315 nm, preferably from 311 to 313 nm, to pass through.

The composition for use according to the invention comprises the combination of the protective compounds diethylhexyl 2,6-naphthalate, diethylamino hydroxybenzoyl hexyl benzoate, phenyl dibenzimidazole tetrasulfonate in weight ratios of 0.9-1.2:0.9:1.2, 0.9:1.2 respectively, preferably of 1:1:1 or 1:1:1.2.

In the composition described herein, the compounds diethylamino hydroxybenzoyl hexyl benzoate and phenyl dibenzimidazole tetrasulfonate have a shielding action on ultraviolet light in the UVA region.

Preferably, the compound phenyl dibenzimidazole tetrasulfonate is present in the composition in the form of a salt, for example a sodium salt.

Advantageously, the combination composition of the invention does not contain an UVB sunscreen.

In the composition for the uses described herein, the compound diethylhexyl 2,6-naphthalate performs a weak filtering action on UVB rays, aiding the formation of the absorption gap or cut-off in the desired UV region with a wavelength from 295 to 320nm.

The combination of the two UVA filtering compounds described above with the compound diethylhexyl 2,6-naphthalate provides broad spectrum protection from ultraviolet radiation exposure by allowing the passage of wavelength radiation in the UVB spectrum range from 295 to 320 nm.

In accordance with some preferred embodiments, the composition described herein comprises the compound diethylhexyl 2,6-naphthalate in an amount from 0.1 to 15% by weight, the compound diethylamino hydroxybenzoyl hexyl benzoate in an amount from 0.1 to 15% by weight, the compound phenyl dibenzimidazole tetrasulfonate, preferably in the form of a salt, for example sodium salt, in an amount of 0.1-15% by weight with respect to the weight of the composition.

In accordance with some embodiments, the compositions described herein may contain further ingredients, in particular antioxidant and/or stabilizing molecules and/or molecules which absorb electromagnetic radiation in the UV despite not being UV filters such as, for example, pentaerythrityl tetra-di-t-butyl hydroxyhydrocinnamate; octadecyl di-t-butyl-4-hydroxyhydrocinnamate; dilauryl thiodipropionate; diethylhexyl siringylidenemalonate; sodium benzotriazolyl phenol sulfonate; sodium benzotriazole, butyl phenol sulfonate; polycrylene, and mixtures thereof.

The composition may also contain one or more shielding substances in the regions of the spectrum other than UV rays, such as the visible, for example in the blue light spectrum with a wavelength range from 380 to 500 nm, optionally extending to the infrared.

According to some embodiments, the compositions described herein may contain hollow spheres of styrene/acrylate copolymer, for example: SunSpheres SPF (Boosters, DOW Personal Care) for example in a percentage comprised between 0.1 and 20%.

Preferably, the hollow spheres of styrene/acrylate copolymer have an outer diameter ranging from 380 to 420 nm, preferably of about 400 nm.

The polymer itself does not absorb ultraviolet radiation, however, the air contained inside the sphere, which has a different refractive index than that of the polymer shell, acts as a dispersion or scattering center for ultraviolet radiation.

In accordance with some embodiments, the composition may further contain one or more ultraviolet radiation filtering compounds, provided that the selected filtering compound, in the amounts present in the composition, does not shield ultraviolet radiation in the cut-off area.

In accordance with further embodiments, the composition may contain salicylic acid derivatives such as ethylhexyl salicylate, homosalate, cinnamic acid derivatives such as ethylhexyl methoxycinnamate, isoamyl p-methoxycinnamate, benzophenone derivatives such as benzophenone-3; benzophenone-4, benzophenone-5, aminobenzoic acid derivatives such as ethylhexyl dimethyl-Paba, PEG-25 Paba; benzimidazole derivatives such as phenylbenzimidazole sulfonic acid, benzoylmethane derivatives such as butylmethoxydibenzoylmethane, isopropyldibenzoylmethane, triazine derivatives such as phenylene bis-diphenyltriazine, diethylhexyl butamido triazone, ethylhexyl triazone, tris-biphenyl triazine, tris-biphenyl triazine (nano), bis-ethylhexyloxyphenol methoxyphenyl triazine, phenyl benzotriazole derivatives such as methylene bis-benzotriazolyl tetramethylbutylphenol, methylene bis-benzotriazolyl tetramethylbutylphenol (nano), drometrizole trisiloxane; diphenyl acrylate derivatives such as Octocrylene, metal oxides such as titanium dioxide, zinc oxide, titanium dioxide (nano), zinc oxide (nano), camphor derivatives such as ecamsule, camphor benzalkonium methosulphate; benzylidene camphorsulfonic acid; polyacrylamidomethylbenzylidene camphor; 4-methylbenzylidene camphor or methoxypropylamino; cyclohexenylidene ethoxyethylcyanoacetate, polysilicone-15, bis-(diethylaminohydroxybenzoylbenzoyl) piperazine (HAA299), and mixtures of these derivatives or compounds.

Advantageously, the compositions described herein filter solar ultraviolet radiation out, while allowing the passage of ultraviolet radiation in an amount suitable for maintaining a physiological production of vitamin D3 and the depletion of the vitamin D3 already formed and present in the epidermis.

Advantageously, the composition does not shield the wavelength radiation in the ultraviolet region between 290 and 315 nm, or it has a partial shielding effect but suitable for converting provitamin D3 (7-dehydrocholesterol, 7-DHC) into previtamin D3 with subsequent isomerization into vitamin D3, thermally induced by body heat. The composition described herein finds application in the treatment of skin diseases with an inflammatory component. These skin diseases include psoriasis, parapsoriasis, atopic dermatitis, diffuse eczematous dermatitis, vitiligo, lichen ruber planus, localized scleroderma, pityriasis rosea, chronic lichenoid pityriasis, rosacea, pityriasis lichenoides et varioliformis acuta (PLEVA), chronic urticaria, eosinophilic pustular folliculitis, polymorphous light eruption, pruritus, prurigo nodularis, acne.

The composition finds specific and preferred application in the phototherapeutic treatment of one or more of the following dermatological diseases:

- atopic dermatitis, inflammatory condition of the skin, with dry itchy skin, desquamation, redness and blistering. For the phototherapeutic treatment of this disease, the application on the skin of a composition which allows ultraviolet radiation of a wavelength between 311 and 313 nm to pass through is particularly suitable.
- vitiligo, a skin disorder characterized by the lack of its characteristic pigment, melanin, which causes the appearance of light spots. For the phototherapeutic treatment of this disease, a composition which allows ultraviolet radiation of a wavelength of 311nm to pass through is particularly suitable.
- acne and rosacea, dermatological diseases that affect a high percentage of subjects of both sexes. For the phototherapeutic treatment of these dermatological diseases, the application on the skin of a composition which allows ultraviolet radiation of a wavelength between 311 and 313 nm to pass through is particularly suitable.
- psoriasis, an inflammatory skin disease of a chronic-relapsing nature which manifests itself in most cases with the appearance of raised erythematous patches or reddened plaques. In its milder forms, it can be treated by phototherapy, in particular with wavelength of 311 nm.

In accordance with another aspect, the invention relates to a pharmaceutical composition comprising an effective amount of a combination composition containing diethylhexyl 2,6-naphthalate, diethylamino hydroxybenzoyl hexyl benzoate, phenyl dibenzimidazole tetrasulfonate, optionally in the form of pharmaceutically acceptable salts, and a pharmaceutically acceptable carrier.

In accordance with some embodiments, the composition for use as described herein has a sun protection factor SPF ranging from 10 to 50+, preferably from 15 to 40. This characteristic is related to the presence of the three protective or filtering compounds which form the core of the combination composition for the uses of the invention.

In another aspect, the invention concerns a non-therapeutic cosmetic use of the composition for topical application to improve the aesthetic appearance of skin affected by skin redness, skin flushing, wherein said composition comprises a combination of the compounds diethylhexyl 2,6-naphthalate, diethylamino hydroxybenzoyl hexyl benzoate, phenyl dibenzimidazole tetrasulfonate and cosmetically acceptable salts thereof, and a cosmetically acceptable carrier for shielding ultraviolet radiation and allowing ultraviolet radiation with wavelengths in the range from 295 to 320 nm to pass through at least partially.

Said cosmetic non-therapeutic use preferably allows to shield ultraviolet radiation and allows ultraviolet radiation with a wavelength in the range from 300 to 315nm, preferably from 311 to 313 nm, to pass through, at least partially.

All the advantageous, preferred embodiments, as well as the advantageous and preferred aspects for the composition for the use of the invention also refer to the non-therapeutic and cosmetic use and are not repeated here.

The sun protection factor, known by the acronym SPF, is an index of the ability of a protective product to interact effectively with solar radiation.

The SPF is a measure of the protection from UVB radiation, UVAPF is an estimate of the protection from UVA radiation, and the critical wavelength ($\lambda$c) is an estimate of the "quality" of this protection.

Typically, the SPF is measured through an *in vivo* test on the skin of volunteer subjects irradiated with increasing doses of UV radiation produced by a lamp, under standardized and highly reproducible conditions.

In particular, the SPF is defined as the ratio between the Minimal Erythema Dose (MED) of the area protected by the solar product and the Minimal Erythema Dose of the unprotected area of the same subject. The SPF is therefore a measure of the solar energy that is needed to produce a sunburn on the protected skin.

The ability of a solar product to protect the skin against UVA radiation can be assessed through three methods, two of which are *in vivo* (IPD, Immediate Pigment Darkening and PPD, Persistent Pigment Darkening) and one is *in vitro* UVAPF. IPD and PPD measure the biological parameter of immediate or permanent pigmentation.

**EP 4 518 849 B1**

Typically, the test for the *in vitro* determination of UVAPF (UNI EN ISO 24443:2012) is based on the evaluation of UV transmittance through a thin layer of product with sunscreen spread on a rough substrate, typically polymethylmethacrylate plates, PMMA.

This test allows to determine the "static" SPF value, the UVAPF value, the SPF/UVAPF ratio, and the critical wavelength of the product under study. The critical lambda of the product is the wavelength at which the area of the spectral absorbance curve represents 90% of the total absorbance.

Typically, satisfactory protection in the UVA-UVB spectrum is achieved when the sunscreen has $\lambda c$ values greater than 370 nm. This value, when combined with the SPF and UVAPF values, makes it possible to evaluate the broad-spectrum quality of the sunscreen.

Similarly, sun filters, which are ingredients having the ability to interact with UV radiation, may be divided into categories according to their ability to interact with ultraviolet radiation.

Filters such as ethylhexyl methoxycinnamate absorb predominantly in the UVB region, so they are used to increase the SPF; filters such as avobenzone absorb in the UVA region and are used to increase the UVAPF.

The composition may contain additional sun filters.

Typically, the compositions described herein contain a physiologically acceptable carrier and are suitable for topical application to the skin or mucous membranes of the human body.

The term "filtering" as used herein, for example referring to a composition for the uses of the invention, refers to a solar composition which filters ultraviolet radiation across a broad spectrum out, while allowing ultraviolet radiation to filter through or pass through when in a selected range of wavelengths from 295 to 320 nm, preferably from 305 to 315.

In the present description, the term "carrier" refers to an excipient, diluent or adjuvant which has no activity and may be present in the composition of the invention. Any carrier and/or excipient suitable for the desired preparation form for administration is contemplated in the uses described herein. Suitable carriers are those commonly used in the cosmetic field in the formulation of solar products for topical application. The terms protective compounds, sunscreen compounds, are intended to mean chemical compounds which, when applied to the skin, act as a filter of sun rays. Advantageously, the combination of sunscreen compounds allows selected sun's rays in a specific range of the UV radiation incident on the skin of an individual in need of treatment to pass or filter through.

In accordance with some embodiments, the composition of the invention contains a pharmaceutically and cosmetically acceptable carrier and the compounds diethylhexyl 2,6-naphthalate, diethylamino hydroxybenzoyl hexyl benzoate, phenyl dibenzimidazole tetrasulfonate, optionally in the form of salts, in an amount in the range from 0.1 to 20% by weight, 0.5 to 15%, 1 to 10% by weight.

Typically, the composition of the invention for cosmetic or pharmaceutical use is in any form suitable for topical/local application and can be in a solid, fluid or semi-fluid form.

Suitable compositions in a solid form include creams, pastes, ointments, gels, bandages for pharmaceutical or cosmetic applications and sticks, make-up.

In accordance with a preferred embodiment, the composition is a cream for topical application.

Suitable compositions in a liquid form include water-based formulations such as suspensions, solutions, lotions, gels.

Suitable compositions in a semi-fluid form include foams and emulsions.

In the composition of the invention, one or more excipients typically used in the base formulation of pharmaceutical or cosmetic formulations can be incorporated, such as oils, glycerin, emollients, emulsifiers, dispersing agents, in amounts which are typical for the desired use.

In the formulation of the composition of the invention additional ingredients may be present, such as bisabolol, inositol, betaine, allantoin, ceramides, for the creamy formulation.

In some embodiments, the fluid or semi-fluid composition of the invention may contain lipophilic substances, such as oils, for example hydrogenated castor oil.

In the case of formulations in a liquid form, water is present as a diluent or solvent, optionally mixed with other liquids used for the formulation of pharmaceutical or cosmetic compositions.

According to another embodiment, the cosmetic composition further comprises one or more of cosmetically acceptable thickeners, solubilizers, preservatives, water, alcohols, glycerin, stabilizers, antioxidants and antibacterials in amounts according to common pharmaceutical or cosmetic practice.

Advantageously, the composition described herein can be formulated as a solar product, having a therapeutic action.

Within the scope set out herein, solar products or sunscreens are intended to mean topical preparations that aim to protect the skin from solar radiation and act as a filter against UVA and/or UVB radiation.

The main purpose of sunscreens or solar products is to intercept solar radiation along the entire spectrum of UV radiation and prevent it from interacting with the skin or skin appendages, damaging them. There are multiple damages resulting from sun exposure and they can affect both the DNA of keratinocytes and the protein material. Furthermore, the cutaneous application of solar products reduces the risk of interaction of UVB rays with the DNA, and of UVA rays with the skin tissue, resulting in the formation of radical species.

The present disclosure is further described below with reference to the following examples representing embodiments of the invention.

## EXAMPLE 1

Emulsion SPF 15 cut off 305-315 nm

| Component (INCI Name) | Amount w/w (%) |
|---|---|
| Disodium Phenyl Dibenzimidazole Tetrasulfonate | 0.10-10.0 |
| Diethylamino Hydroxybenzoyl Hexyl Benzoate | 0.10-10.0 |
| Diethylhexyl 2,6-Naphtalate | 0.10-10.0 |
| PEG-100 Stearate | 0.10-4.0 |
| Glyceryl Stearate | 0.10-6.0 |
| Cetearyl Alcohol | 0.10-4.0 |
| Glycerin | 1.0-6.0 |
| Disodium EDTA | 0.050-0.30 |
| Cocoyl Adipic Acid/Trimethylolpropane Copolymer | 2.50-7.5 |
| Dibutyl Adipate | 2.50-7.5 |
| Phenylpropanol | 0.10-1.0 |
| Propanediol | 0.10-1.0 |
| Caprylyl Glycol | 0.10-1.0 |
| Sodium hydroxide solution 30% | 0.05-5.0 |
| Citric acid | 0.002-0.5 |
| Aqua | *q.s.* to 100.00 |

## EXAMPLE 2 (cut off)

Emulsion SPF 30

| Component (INCI Name) | Amount w/w (%) |
|---|---|
| Disodium Phenyl Dibenzimidazole Tetrasulfonate | 0.10-10.0 |
| Diethylamino Hydroxybenzoyl Hexyl Benzoate | 0.10-10.0 |
| Diethylhexyl 2,6-Naphtalate | 0.10-10.0 |
| Methylene Bis-Benzotriazolyl Tetramethylbutylphenol (Nano) | 0.10-5.0 |
| Decyl glucoside | 05-2.50 |
| Propylene glyco | 0.005-3.0 |
| Xanthan gum | 0.005-0.5 |
| PEG-100 Stearate | 0.10-4.0 |
| Glyceryl Stearate | 0.10-6.0 |
| Cetearyl alcohol | 0.10-3.0 |
| Glycerin | 1.00-6.0 |
| Disodium EDTA | 0.050-0.30 |
| Cocoyl Adipic Acid/Trimethylolpropane Copolyme | 2.50-7.5 |
| Dibutyl Adipate | 2.50-7.5 |
| Phenylpropanol | 0.10-1.0 |
| Propanediol | 0.10-1.0 |
| Caprylyl glycol | 0.10-1.0 |
| Sodium hydroxide solution 30% | 0.05-5.0 |

(continued)

| Component (INCI Name) | Amount w/w (%) |
|---|---|
| Citric acid | 002-0.5 |
| Aqua | *q.s.* 100.00 |

## EXAMPLE 3

Emulsion SPF 30

| Component (INCI Name) | Amount w/w (%) |
|---|---|
| Disodium Phenyl Dibenzimidazole Tetrasulfonate | 0.10-10.0 |
| Diethylamino Hydroxybenzoyl Hexyl Benzoate | 0.10-10.0 |
| Diethylhexyl 2,6-Naphtalate | 0.10-10.0 |
| Methylene Bis-Benzotriazolyl Tetramethylbutylphenol (Nano) | 0.10-5.0 |
| Decyl Glucoside | 0.05-2.50 |
| Propylene Glycol | 0.005-3.0 |
| Xanthan Gum | 0.005-0.5 |
| PEG-100 Stearate | 0.10-4.0 |
| Glyceryl Stearate | 0.10-6.0 |
| Cetearyl Alcohol | 0.10-3.0 |
| Glycerin | 1.00-6.0 |
| Disodium EDTA | 0.050-0.30 |
| Cocoyl Adipic Acid/Trimethylolpropane Copolymer | 2.50-7.5 |
| Butyloctyl Salicylate | 0.50-3.0 |
| Dibutyl Adipate | 2.50-7.5 |
| Phenylpropanol | 0.10-1.0 |
| Propanediol | 0.10-1.0 |
| Caprylyl Glycol | 0.10-1.0 |
| Sodium Hydroxide Solution 30% | 0.05-5.0 |
| Citric acid | 0.002-0.5 |
| Aqua | *q.s.* 100.00 |

## EXAMPLE 4A

Two compositions for the uses of the invention were tested to verify the effective formation of a gap (cut-off) in the wavelength range from 295 to 320 nm.

The two formulations had different excipients but identical ratios of the active ingredients: Diethylhexyl 2,6-naphthalate: diethylamino hydroxybenzoyl hexyl benzoate: disodium phenyl dibenzimidazole tetrasulfonate = 1:1:1.

In the composition PT0834-A20 the disodium phenyl dibenzimidazole tetrasulfonate was inserted in a special hydrotalcite.

Composition PT0834-A19

| Component (INCI Name) | Amount w/w (%) |
|---|---|
| Disodium Phenyl Dibenzimidazole Tetrasulfonate | 3.0-9.0 |
| Diethylamino Hydroxybenzoyl Hexyl Benzoate | 3.0-9.0 |
| Diethylhexyl 2,6-Naphtalate | 3.0-9.0 |
| Zinc/Aluminium/Hydroxide Hydrotalcite | 8.0-13.0 |
| PEG-100 Stearate | 0.10-4.0 |

(continued)

| Component (INCI Name) | Amount w/w (%) |
|---|---|
| Glyceryl Stearate | 0.10-7.0 |
| Cetearyl Alcohol | 0.10-4.0 |
| Disodium EDTA | 0.050-0.30 |
| Propanediol | 0.1-5.0 |
| Cocoyl Adipic Acid/Trimethylolpropane Copolymer | 2.50-7.5 |
| Dibutyl Adipate | 2.50-7.5 |
| Sodium Hydroxide Solution 30% | 0.05-5.0 |
| Phenoxyethanol | 0.10-1.0 |
| o-Cymen-5-ol | 0.02-0.10 |
| Caprylhydroxamic acid | 0.02-1.0 |
| Citric acid | *q.s.* |
| Aqua | *q.s.* to 100.00 |

Composition PT0834-A20

| Component (INCI Name) | Amount w/w (%) |
|---|---|
| Disodium Phenyl Dibenzimidazole Tetrasulfonate | 3.0-9.0 |
| Diethylamino Hydroxybenzoyl Hexyl Benzoate | 3.0-9.0 |
| Diethylhexyl 2,6-Naphtalate | 3.0-9.0 |
| PEG-100 Stearate | 0.10-4.0 |
| Glyceryl Stearate | 0.10-7.0 |
| Cetearyl Alcohol | 0.10-4.0 |
| Disodium EDTA | 0.050-0.30 |
| Propanediol | 0.1-5.0 |
| Cocoyl Adipic Acid/Trimethylolpropane Copolymer | 2.50-7.5 |
| Dibutyl Adipate | 2.50-7.5 |
| Sodium Hydroxide Solution 30% | 0.05-5.0 |
| Phenoxyethanol | 0.10-1.0 |
| o-Cymen-5-ol | 0.02-0.10 |
| *C*aprylhydroxamic acid | 0.02-1.0 |
| Citric acid | *q.s.* |
| Aqua | *q.s.* to 100.00 |

For determining the SPF *in vitro,* the method of Brian L. Diffey e J. Robson del 1989 (J. Soc. Cosmet. Chem., 40, 127-133 (May/June 1989), A new substrate to measure sunscreen protection factors throughout the ultraviolet spectrum, B. L. DIFFEY and J. ROBSON, Regional Medical Physics Department Dryburn Hospital Durham DH1 5TW, U.K.) was used.

This method is based on the measurement of the spectral transmittance of a product, i.e. the ratio between the radiation transmitted by the sample and the total incident radiation.

Instruments: Jasco V730 UV-Vis spectrophotometer, Leukofix plaster, analytical balance, laboratory glassware.

By applying a known amount of product on a suitable substrate with a predetermined surface, so as to obtain a homogeneous translucent film, the monochromatic spectral transmittance T($\lambda$) is obtained:

$$T(\lambda): T(\lambda)=Ss(\lambda)/S0(\lambda),$$

wherein:

Ss ($\lambda$) is the transmittance of the substrate at wavelength $\lambda$;
S0 ($\lambda$) is the transmittance of the substrate when the sample was stratified on it.

By quantifying this value every 0.5 nm, across the total range of wavelengths (290-400 nm), the SPF value of the product is obtained, as given by the equation:

$$SPF = \frac{\sum\limits_{290nm}^{400nm} E(\lambda)\varepsilon(\lambda)}{\sum\limits_{290nm}^{400nm} E(\lambda)\varepsilon(\lambda)/PF(\lambda)}$$

wherein
E($\lambda$) is the spectral irradiance of sunlight. B($\lambda$) is the erythematous action spectrum of solar radiation.
The analysis with this method allows to obtain three different values:
UVA/UVB Ratio.
Sun Protection Factor (SPF).
UV Protection Factor.

Procedure

The analyses were carried out using the Diffey-Robson method, which involves the use of a Leukofix surgical tape, sufficiently transparent to UV-Vis light and able to simulate the human epidermis texture. A UV-Vis JASCO mod. V-730PC spectrophotometer was used to determine the transmittance.
The work protocol adopted by the company internal laboratory includes:

- surface of 5 cm x 5 cm size
- applied dose equal to $2.00\pm0.04$ mg/cm$^2$

For each formulation, at least 3 supports are prepared and analyzed, for each of which 6 scans are performed in different portions of the surface of the patch itself. This methodology allows to guarantee a good reproducibility of the obtained data.
The analysis results are reported in the graph of Figure 1 which shows the absorbance curves at different wavelengths of the two tested compositions. From the two curves, it can be seen that the absorbance of both compositions rises progressively for wavelengths lower than 295 nm and higher than 320 nm, while there is a hollow in the curve in the wavelength range from 295 to 320 nm, in particular from 300 to 315 nm, values consistent with those of the invention.
The SPF measured for PT0834-A19 was 12.53 against 15.60 for the formulation PT0834-A20.

EXAMPLE 4B

Two formulations according to the invention were tested, PT0834-A21 and PT0834-A22, having the same formulation of Example 4A but with a higher amount of Disodium Phenyl Dibenzimidazole Tetrasulfonate, while keeping the amount of the other two compounds unchanged.
In particular, the two compositions contained the filtering compounds in the following ratios: Diethylhexyl 2,6-Naphtalate: Diethylamino Hydroxybenzoyl Hexyl Benzoate: Disodium Phenyl Dibenzimidazole Tetrasulfonate = 1:1:1.2. The compositions (PT0834-A21 and PT0834-A22) were analyzed using the Diffey-Robson methodology and compared with two SPF 15 formulations having a SPF/UVAPF ratio 1/3 found on the market, COMM1 SPF15 and COMM2 SPF15.
The results of the comparative tests are illustrated in the graphs of Figure 2 showing the absorbance curves at different wavelengths of the four tested compositions. The curves show how the two compositions of the invention, PT0834-A21 and PT0834-A22, have a decay of the ultraviolet radiation absorption in the region from 295 to 320 nm, in particular from 300 to 315 nm, values which are consistent with those of the invention.
Conversely, the two products found on the market do not have a gap in the UVB region of interest for the phototherapeutic treatment described herein.
The data collected during the test for the samples tested in Examples 4A and 4B, relating to Sun Protection Factor (SPF), UVA Protection Factor (UVA-PF), critical lambda, UVA-PF/SPF ratio, shielded UVA percentage, shielded UVB percentage and UVA/UVB ratio are shown in the following Table.

| Parameters | COMM1 | | PT0834-A22 | | PT0834-A21 | | COMM2 | | PT0834-A19 | | PT0834-A20 | |
|---|---|---|---|---|---|---|---|---|---|---|---|---|
| | Value | Stand. Dev. | Value | Stand. Dev. | Value | Stand. Dev. | Value | Stand. Dev | Value | Stand. Dev | Value | Stand. Dev |
| Sun Protection Factor | 38.5 | 7.61 | 12.76 | 2.55 | 14.35 | 2.87 | 22.86 | 4.57 | 12.53 | 2.51 | 15.60 | 3.12 |
| UVA_PF | 14.82 | 2.96 | 16.32 | 3.26 | 21.05 | 4.21 | 16.31 | 3.26 | 19.18 | 3.84 | 24.47 | 4.89 |
| Critical Lambda | 375.80 | 3.00 | 384.40 | 3.00 | 382.00 | 3.00 | 379.80 | 3.00 | 385.00 | 3.00 | 381.00 | 3.00 |
| UVA-PF/SPF Ratio | 0.39 | 0.11 | 1.28 | 0.36 | 1.47 | 0.41 | 0.71 | 0.20 | 1.53 | 0.43 | 1.57 | 0.44 |
| Shielded UVA (%) | 90.76 | 0.02 | 92.28 | 0.02 | 92.65 | 0.02 | 91.94 | 0.02 | 93.62 | 0.02 | 92.94 | 0.02 |
| Shielded UVB (%) | 99.15 | 0.02 | 92.52 | 0.02 | 93.77 | 0.02 | 97.90 | 0.02 | 92.66 | 0.02 | 94.32 | 0.02 |
| UVA/UVB | 0.92 | 0.00 | 1.00 | 0.00 | 0.99 | 0.00 | 0.94 | 0.00 | 1.01 | 0.00 | 0.99 | 0.00 |

The following tests were carried out with the aim of reaching higher SPFs without canceling the cut-off.

Of the two commercial SPF 15 emulsions (COMM1 and COMM2) of reference, COMM 1 has SPF values consistent with the product declaration, while COMM2 shows values higher than expected, where the SPF value is around 38. This discrepancy is due to the presence of nanometric titanium oxide, which generates scattering phenomena in the UVB region, which cannot be integrated by the instrument.

EXAMPLE 5

The absorbance and transmittance spectra at different wavelengths (240-400 nm) of four samples of the invention (PT0834 T-20-1/2/3) and of a commercial product sample (COMM3) with SPF 1/3 - SPF/UVAPF $\geq$ 1/3, critical lambda > 780 nm, were measured.

Composition PT0834-T20-1

Emulsion SPF 30 cut-off 305-315 nm

| Component (INCI Name) | Amount w/w (%) |
| --- | --- |
| Disodium Phenyl Dibenzimidazole Tetrasulfonate | 3.0-9.0 |
| Diethylamino Hydroxybenzoyl Hexyl Benzoate | 3.0-9.0 |
| Diethylhexyl 2,6-Naphtalate | 3.0-9.0 |
| Butyloctyl Salicylate | 4.0-7.5 |
| PEG-100 Stearate | 0.10-4.0 |
| Glyceryl Stearate | 0.10-7.0 |
| Cetearyl Alcohol | 0.10-4.0 |
| Glycerin | 1.0-6.0 |
| Disodium EDTA | 0.050-0.30 |
| Methylene Bis-Benzotriazolyl Tetramethylbutylphenol (Nano) | 2.5-10.0 |
| Decyl Glucoside | 0.05-4.50 |
| Propylene Glycol | 0.01-1.0 |
| Xanthan Gum | 0.005-1.0 |
| Cocoyl Adipic Acid/Trimethylolpropane Copolymer | 2.50-7.5 |
| Dibutyl Adipate | 2.50-7.5 |
| Sodium Hydroxide Solution 30 | 0.05-5.0 |
| Phenoxyethanol | 0.10-1.0 |
| o-Cymen-5-ol | 0.02-0.10 |
| Citric Acid | *q.s.* |
| Aqua | *q.s.* to 100.00 |

Composition PT0834-T20-2

| Component (INCI Name) | Amount w/w (%) |
| --- | --- |
| Disodium Phenyl Dibenzimidazole Tetrasulfonate | 3.0-9.0 |
| Diethylamino Hydroxybenzoyl Hexyl Benzoate | 3.0-9.0 |
| Diethylhexyl 2,6-Naphtalate | 3.0-9.0 |
| PEG-100 Stearate | 0.10-4.0 |
| Glyceryl Stearate | 0.10-7.0 |
| Cetearyl Alcohol | 0.10-4.0 |
| Glycerin | 1.0-6.0 |
| Disodium EDTA | 0.050-0.30 |
| Bis-Ethylhexyloxyphenol Methoxyphenyl Triazine | 2.5-10.0 |

(continued)

| Component (INCI Name) | Amount w/w (%) |
|---|---|
| Cocoyl Adipic Acid/Trimethylolpropane Copolymer | 2.50-7.5 |
| Dibutyl Adipate | 2.50-7.5 |
| Sodium Hydroxide Solution 30 % | 0.05-5.0 |
| Phenoxyethanol | 0.10-1.0 |
| o-Cymen-5-ol | 0.02-0.10 |
| Citric acid | *q.s.* |
| Aqua | *q.s.* to 100.00 |

Composition PT0834-T20-3

| Component (INCI Name) | Amount w/w (%) |
|---|---|
| Disodium Phenyl Dibenzimidazole Tetrasulfonate | 3.0-9.0 |
| Diethylamino Hydroxybenzoyl Hexyl Benzoate | 3.0-9.0 |
| Diethylhexyl 2,6-Naphtalate | 3.0-9.0 |
| PEG-100 Stearate | 0.10-4.0 |
| Glyceryl Stearate | 0.10-7.0 |
| Cetearyl Alcohol | 0.10-4.0 |
| Glycerin | 1.0-6.0 |
| Disodium EDTA | 0.050-0.30 |
| Methylene Bis-Benzotriazolyl Tetramethylbutylphenol (Nano) | 2.5-10.0 |
| Decyl Glucoside | 0.05-4.50 |
| Propylene Glycol | 0.01-1.0 |
| Xanthan Gum | 0.005-1.0 |
| Cocoyl Adipic Acid/Trimethylolpropane Copolymer | 2.50-7.5 |
| Dibutyl Adipate | 2.50-7.5 |
| Sodium hydroxide solution 30 | 0.05-5.0 |
| Phenoxyethanol | 0.10-1.0 |
| o-Cymen-5-ol | 0.02-0.10 |
| Citric acid | *q.s.* |
| Aqua | to 100.00 |

Product COMM3

The commercial product identified by the acronym COMM3 had the following composition:
Aqua, C12-15 Alkyl Benzoate, Glycerin, Ethylhexyl Salicylate, Styrene/Acrylate Copolymer, Dimethicone, Octocrylene, Alcohol Denat., Butyl Methoxydibenzoylmethane, Pentylene Glycol, Bis-Ethylhexyloxyphenol Methoxyphenyl Triazine, Potassium Cetyl Phosphate, Propylene Glycol, Synthetic Wax, Caprylyl Glycol, Dimethiconol, Disodium EDTA, Drometrizole Trisiloxane, Ethylhexyl Triazone, Glyceryl Stearate, Hydroxypropyl Methylcellulose, Isopropyl Lauroyl Sarcosinate, Palmitic Acid, PEG-100 Stearate, PEG-8 Laurate, Phenoxyethanol, Sodium Polyacrylate, Stearic Acid, Terephthalylidene Dicamphor Sulfonic Acid, Tocopherol, Triethanolamine.

The results are depicted in the enclosed Figures 3-6, as previously illustrated.

The analyses were carried out *in vitro,* with the Diffey-Robson method *(In vitro* determination of the sun protection factor (SPF) of a cosmetic product using a spectrophotometer and ISR-2200 integrating sphere).

In the method, PMMA (polymethylmethacrylate, Schonberg) plates were used. PMMA plates are manufactured from a polymethylmethacrylate sheet. A plate surface is roughened in a standardized manner (sandblasting under controlled conditions). In the present study, an ISR-2200 Integrating Sphere (Shimadzu Italy s.r.l.) was used. The integrating sphere is an optical device capable of collecting light transmitted in all directions after passing through a substrate. The integrating sphere is used as a diffuse light collector.

The solar product was applied on the rough surface of the PMMA plate in an amount of 1.3 mg/cm$^2$.

The solar product is deposited in small droplets of approximately equal volume, evenly distributed over the entire surface of the plate.

Immediately after its application, the product is spread over the entire surface of the plate. The sample is then left for at least 15 minutes in the dark at room temperature, in order to facilitate the formation of a stabilized product film. The blank is obtained by reading an untreated PMMA plate.

For each sample, 5 readings are taken.

$$SPF = \frac{\sum\limits_{290nm}^{400nm} E(\lambda)\varepsilon(\lambda)}{\sum\limits_{290nm}^{400nm} E(\lambda)\varepsilon(\lambda)/PF(\lambda)}$$

wherein:

E: spectral erythema efficacy (Figures 2, 3 and Table 1)
$\varepsilon$: spectral solar irradiance (Figures 2, 3 and Table 1)
PF: Tb/Tc (Tb: transmittance of the blank, Tc: transmittance of the sample)

Test/Measurement Results

The formulation "COMM3", had an SPF in line with commercial sunscreen products but showed an almost linear absorption in the UVB region between 390 and 315 nm, as shown in Figure 3. The tests did not show any curve valley in the wavelength region of interest from 290 to 315 nm.

This formulation was not found to be suitable for phototherapy uses according to the present invention.

The composition "PT0834-T20-1", had an SPF in line with commercial sunscreen products. The tests showed a curve valley in the wavelength region of interest from 305 to 315 nm, as illustrated in the enclosed Figure 4.

This formulation was found to be suitable for uses in phototherapy according to the present invention.

The composition "PT0834-T20-2", had an SPF of 15. The tests showed a curve valley in the wavelength region of interest from 305 to 310 nm, as illustrated in the attached Figure 5.

This composition was found to be suitable for uses in phototherapy according to the present invention.

The composition "PT0834-T20-3", had an SPF of 50. The tests highlighted a curve valley in the wavelength region of interest from 305 to 315 nm, as illustrated in the enclosed Figure 6.

This composition was found to be suitable for uses in phototherapy according to the present invention.

**Claims**

1. A topical composition filtering ultraviolet rays for use in the phototherapeutic treatment of a skin disease with an inflammatory component comprising a combination of compounds that protect the skin from ultraviolet radiation while allowing UVB radiation with a wavelength in the range from 295 to 320 nm to pass through, wherein said combination of protective compounds comprises diethylhexyl 2,6-naphthalate, diethylamino hydroxybenzoyl hexyl benzoate, phenyl dibenzimidazole tetrasulfonate, and pharmaceutically acceptable salts thereof, wherein said skin disease with inflammatory component is selected from the group consisting of psoriasis, atopic dermatitis, acne, acne inversa/hidradenitis suppurativa, rosacea, seborrheic dermatitis, eczematous dermatitis, vitiligo, folliculitis, pityriasis rosea, chronic lichenoid pityriasis, pityriasis lichenoides et varioliformis acuta, chronic urticaria, eosinophilic pustular folliculitis, polymorphous light eruption, pruritus and prurigo nodularis.

2. The composition for use according to claim 1, wherein the protective compounds allow UVB radiation with a wavelength in the range from 305 to 315 nm, preferably from 311 to 313 nm, to pass through.

3. The composition for use according to claim 1 or 2, wherein the combination of protective compounds comprises diethylhexyl 2,6-naphthalate, diethylamino hydroxybenzoyl hexyl benzoate, phenyl dibenzimidazole tetrasulfonate in ratios by weight of 0.9-1.2:0.9:1.2, 0.9:1.2, respectively, preferably of 1:1:1 or 1:1:1.2.

4. The composition for use according to anyone of claims 1-3, wherein the phototherapeutic treatment of the skin comprises the exposure of the skin of an individual in need of treatment to ultraviolet rays for a period of time from 15 minutes to 4 hours, preferably from thirty minutes to one hour.

5.  The composition for use according to anyone of claims 1-4, wherein the phototherapeutic treatment comprises the application of the composition on the skin in an amount ranging from 1.5 to 2.5 mg/cm$^2$, preferably from 1.8 to 2.2 mg/cm$^2$, more preferably of 2 mg/cm$^2$.

6.  The composition for use according to anyone of claims 1-5, wherein said composition comprises a further protective or filtering compound selected from the group consisting of ethylhexyl salicylate, homosalate, cinnamic acid derivatives selected from ethylhexyl methoxycinnamate, isoamyl p-methoxycinnamate, benzophenone derivatives selected from benzophenone-3, benzophenone-4, benzophenone-5, aminobenzoic acid derivatives selected from ethylhexyl dimethyl Paba, PEG-25 Paba, benzimidazole derivatives selected from phenylbenzimidazole sulfonic acid, benzoylmethane derivatives selected from butyl methoxydibenzoylmethane, isopropyl dibenzoylmethane, triazine derivatives selected from phenylene bis-diphenyltriazine, diethylhexyl butamido triazone, ethylhexyl triazone, tris-biphenyl triazine, tris-biphenyl triazine (nano), bis-ethylhexyloxyphenol methoxyphenyl triazine, phenyl benzotriazole derivatives selected from methylene bis-benzotriazolyl tetramethylbutylphenol, methylene bis-benzotriazolyl tetramethylbutylphenol (nano), diphenyl acrylate derivatives selected from octocrylene, metal oxides selected from Titanium Dioxide, Zinc Oxide, Titanium Dioxide (nano), Zinc Oxide (nano), camphor derivatives selected from Camphor benzalkonium methosulfate; benzylidene camphor sulfonic acid; polyacrylamidomethyl benzylidene camphor; 4-methylbenzylidene camphor or methoxypropylamino cyclohexenylidene ethoxyethylcyanoacetate, polysilicone-15, bis-(diethylaminohydroxybenzoylbenzoyl) piperazine (HAA299) and mixtures of these derivatives and compounds.

7.  The composition for use according to anyone of claims 1-6, wherein said composition has a sun protection factor ranging from 10 to 50+, preferably from 30 to 50.

8.  The composition for use according to anyone of claims 1-7, wherein the compound diethylhexyl 2,6-naphthalate is in an amount from 0.1 to 15% by weight, the compound diethylamino hydroxybenzoyl hexyl benzoate is in an amount from 0.1 to 15% by weight, the compound phenyl dibenzimidazole tetrasulfonate, preferably in the form of salt, for example sodium salt, is in an amount from 0.1 to 15% by weight with respect to the total weight of the composition.

9.  The composition for use according to anyone of claims 1-8, wherein said composition has a sun protection factor ranging from 15 to 30, preferably from 15-20.

10. The composition for use according to anyone of claims 1-9, wherein said composition does not include UVB filters.

11. A non-therapeutic cosmetic use of a composition for topical application to improve the aesthetic appearance of skin affected by skin redness, skin flushing, wherein said composition comprises a combination of the compounds diethylhexyl 2,6-naphthalate, diethylamino hydroxybenzoyl hexyl benzoate, phenyl dibenzimidazole tetrasulfonate and cosmetically acceptable salts thereof, and a cosmetically acceptable carrier for shielding ultraviolet radiation, and allowing ultraviolet radiation having a wavelength in the range from 295 to 320 nm to pass through, at least partially.

12. The cosmetic non-therapeutic use according to claim 11, wherein said use is for shielding ultraviolet radiations and allowing ultraviolet radiations with wavelengths in the range from 300 to 315 nm, preferably from 311 at 313 nm, to pass through, at least partially.

**Patentansprüche**

1.  Topische Zusammensetzung, die ultraviolette Strahlen filtert, zur Verwendung bei der phototherapeutischen Behandlung einer Hauterkrankung mit entzündlicher Komponente, umfassend eine Kombination von Verbindungen, die die Haut vor ultravioletter Strahlung schützen, während UVB-Strahlung mit einer Wellenlänge im Bereich von 295 bis 320 nm durchgelassen wird, wobei die Kombination von Schutzverbindungen Diethylhexyl-2,6-naphthalat, Diethylaminohydroxybenzoylhexylbenzoat, Phenyldibenzimidazoltetrasulfonat und pharmazeutisch verträgliche Salze davon umfasst, wobei die Hauterkrankung mit entzündlicher Komponente ausgewählt ist aus der Gruppe, bestehend aus Psoriasis, atopischer Dermatitis, Akne, Akne inversa/Hidradenitis suppurativa, Rosacea, seborrhoischer Dermatitis, ekzematöser Dermatitis, Vitiligo, Follikulitis, Pityriasis rosea, chronischer Pityriasis lichenoides, Pityriasis lichenoides et varioliformis acuta, chronischer Urtikaria und eosinophiler pustulöser Follikulitis, polymorpher Lichtdermatose, Pruritus und Prurigo nodularis.

2. Zusammensetzung zur Verwendung nach Anspruch 1, wobei die Schutzverbindungen UVB-Strahlung mit einer Wellenlänge im Bereich von 305 bis 315 nm, vorzugsweise von 311 bis 313 nm, durchlassen.

3. Zusammensetzung zur Verwendung nach Anspruch 1 oder 2, wobei die Kombination von Schutzverbindungen Diethylhexyl-2,6-naphthalat, Diethylaminohydroxybenzoylhexylbenzoat, Phenyldibenzimidazoltetrasulfonat in Gewichtsverhältnissen von 0,9-1,2 : 0,9 : 1,2 bzw. 0,9 : 1,2, vorzugsweise von 1 : 1 : 1 oder 1 : 1 : 1,2, umfasst.

4. Zusammensetzung zur Verwendung nach einem der Ansprüche 1-3, wobei die phototherapeutische Behandlung der Haut die Bestrahlung der Haut einer behandlungsbedürftigen Person mit ultravioletten Strahlen für einen Zeitraum von 15 Minuten bis 4 Stunden, vorzugsweise von dreißig Minuten bis zu einer Stunde, umfasst.

5. Zusammensetzung zur Verwendung nach einem der Ansprüche 1-4, wobei die phototherapeutische Behandlung das Auftragen der Zusammensetzung auf die Haut in einer Menge im Bereich von 1,5 bis 2,5 mg/cm$^2$, vorzugsweise von 1,8 bis 2,2 mg/cm$^2$, besonders bevorzugt von 2 mg/cm$^2$, umfasst.

6. Zusammensetzung zur Verwendung nach einem der Ansprüche 1-5, wobei die Zusammensetzung eine weitere Schutz- oder Filterverbindung umfasst, ausgewählt aus der Gruppe, bestehend aus Ethylhexylsalicylat, Homosalat, Zimtsäurederivaten, ausgewählt aus Ethylhexylmethoxycinnamat, Isoamyl-p-methoxycinnamat, Benzophenonderivaten, ausgewählt aus Benzophenon-3, Benzophenon-4, Benzophenon-5, Aminobenzoesäurederivaten, ausgewählt aus Ethylhexyldimethyl-PABA, PEG-25-PABA, Benzimidazolderivate, ausgewählt aus Phenylbenzimidazolsulfonsäure, Benzoylmethanderivate, ausgewählt aus Butylmethoxydibenzoylmethan, Isopropyldibenzoylmethan, Triazinderivate, ausgewählt aus Phenylen-bis-diphenyltriazin, Diethylhexylbutamidotriazon, Ethylhexyltriazon, Tris-biphenyltriazin, Tris-biphenyltriazin (nano), Bisethylhexyloxyphenolmethoxyphenyltriazin, Phenylbenzotriazolderivate, ausgewählt aus Methylen-bis-benzotriazolyltetramethylbutylphenol, Methylen-bis-benzotriazolyltetramethylbutylphenol (nano), Diphenylacrylatderivate, ausgewählt aus Octocrylen, Metalloxide, ausgewählt aus Titandioxid, Zinkoxid, Titandioxid (nano), Zinkoxid (nano), Campherderivate, ausgewählt aus Campherbenzalkoniummethosulfat; Benzylidencamphersulfonsäure; Polyacrylamidomethylbenzylidencampher; 4-Methylbenzylidencampher oder Methoxypropylaminocyclohexenylidenethoxyethylcyanoacetat, Polysilicon-15, Bis-(diethylaminohydroxybenzoylbenzoyl)piperazin (HAA299) und Mischungen dieser Derivate und Verbindungen.

7. Zusammensetzung zur Verwendung nach einem der Ansprüche 1-6, wobei die Zusammensetzung einen Lichtschutzfaktor von 10 bis 50+, vorzugsweise von 30 bis 50, aufweist.

8. Zusammensetzung zur Verwendung nach einem der Ansprüche 1-7, wobei die Verbindung Diethylhexyl-2,6-naphthalat in einer Menge von 0,1 bis 15 Gew.-% vorliegt, die Verbindung Diethylaminohydroxybenzoylhexylbenzoat in einer Menge von 0,1 bis 15 Gew.-% vorliegt, die Verbindung Phenyldibenzimidazoltetrasulfonat, vorzugsweise in Form eines Salzes, beispielsweise eines Natriumsalzes, in einer Menge von 0,1 bis 15 Gew.-%, bezogen auf das Gesamtgewicht der Zusammensetzung, vorliegt.

9. Zusammensetzung zur Verwendung nach einem der Ansprüche 1-8, wobei die Zusammensetzung einen Lichtschutzfaktor von 15 bis 30, vorzugsweise von 15-20, aufweist.

10. Zusammensetzung zur Verwendung nach einem der Ansprüche 1-9, wobei die Zusammensetzung keine UVB-Filter beinhaltet.

11. Nicht-therapeutische kosmetische Verwendung einer Zusammensetzung zur topischen Anwendung zur Verbesserung des ästhetischen Erscheinungsbildes einer von Hautrötungen und Flush betroffenen Haut, wobei die Zusammensetzung eine Kombination der Verbindungen Diethylhexyl-2,6-naphthalat, Diethylaminohydroxybenzoylhexylbenzoat, Phenyldibenzimidazoltetrasulfonat und kosmetisch verträglichen Salzen davon sowie einen kosmetisch verträglichen Träger zum Abschirmen ultravioletter Strahlung umfasst und ultraviolette Strahlung mit einer Wellenlänge im Bereich von 295 bis 320 nm zumindest teilweise durchlässt.

12. Kosmetische nicht-therapeutische Verwendung nach Anspruch 11, wobei die Verwendung zum Abschirmen ultravioletter Strahlung und zum zumindest teilweisen Durchlassen von ultravioletter Strahlung mit Wellenlängen im Bereich von 300 bis 315 nm, vorzugsweise von 311 bis 313 nm, dient.

**Revendications**

1. Composition topique filtrant les rayons ultraviolets pour une utilisation dans le traitement photothérapeutique d'une maladie de la peau avec un composant inflammatoire comprenant une combinaison de composés qui protègent la peau contre le rayonnement ultraviolet tout en laissant passer le rayonnement UVB avec une longueur d'onde dans la plage de 295 à 320 nm, dans laquelle ladite combinaison de composés protecteurs comprend le 2,6-naphtalate de diéthylhexyle, le benzoate de diéthylamino hydroxybenzoyl hexyle, le tétrasulfonate de phényl dibenzimidazole, et leurs sels pharmaceutiquement acceptables, dans laquelle ladite maladie de la peau avec composant inflammatoire est choisie dans le groupe constitué par le psoriasis, la dermatite atopique, l'acné, l'acné inverse/hidradénite suppurée, la rosacée, la dermatite séborrhéique, la dermatite eczémateuse, le vitiligo, la folliculite, le pityriasis rosea, le pityriasis lichénoïde chronique, le pityriasis lichenoides et varioliformis acuta, l'urticaire chronique, la folliculite pustulaire eosinophile, l'éruption de lumière polymorphe, le pruritus et le nodulus.

2. Composition à utiliser selon la revendication 1, dans laquelle les composés protecteurs permettent le passage du rayonnement UVB avec une longueur d'onde dans la plage de 305 à 315 nm, de préférence de 311 à 313 nm.

3. Composition à utiliser selon la revendication 1 ou 2, dans laquelle la combinaison de composés protecteurs comprend le 2,6-naphtalate de diéthylhexyle, le benzoate de diéthylamino hydroxybenzoyl hexyle, le tétrasulfonate de phényl dibenzimidazole dans des rapports en poids de 0,9-1,2:0,9:1,2, 0,9:1,2, respectivement, de préférence de 1:1:1 ou 1:1:1,2.

4. Composition à utiliser selon l'une quelconque des revendications 1 à 3, dans laquelle le traitement photothérapeutique de la peau comprend l'exposition de la peau d'un individu ayant besoin d'un traitement aux rayons ultraviolets pendant une période de temps de 15 minutes à 4 heures, de préférence de trente minutes à une heure.

5. Composition à utiliser selon l'une quelconque des revendications 1 à 4, dans laquelle le traitement photothérapeutique comprend l'application de la composition sur la peau en une quantité allant de 1,5 à 2,5 mg/cm$^2$, de préférence de 1,8 à 2,2 mg/cm$^2$, plus préférablement égale à 2 mg/cm$^2$.

6. Composition à utiliser selon l'une quelconque des revendications 1 à 5, dans laquelle ladite composition comprend un composé protecteur ou filtrant supplémentaire choisi parmi le groupe constitué de salicylate d'éthylhexyle, d'homosalate, de dérivés de l'acide cinnamique choisis parmi le méthoxycinnamate d'éthylhexyle, le p-méthoxycinnamate d'isoamyle, de dérivés de benzophénone choisis parmi la benzophénone-3, la benzophénone-4, la benzophénone-5, de dérivés de l'acide aminobenzoïque choisis parmi l'éthylhexyl diméthyl PABA, le PEG-25 PABA, de dérivés de benzimidazole choisis parmi l'acide phénylbenzimidazole sulfonique, de dérivés de benzoylméthane choisis parmi le butyl méthoxydibenzoylméthane, l'isopropyl dibenzoylméthane, de dérivés de triazine choisis parmi le phénylène bis-diphényltriazine, le diéthylhexyl butamido triazone, l'éthylhexyl triazone, la tris-biphényl triazine, la tris-biphényl triazine (nano), la bis-éthylhexyloxyphénol méthoxyphényl triazine, de dérivés de phényl benzotriazole sélectionnés parmi le méthylène bis-benzotriazolyl tétraméthylbutylphénol, le méthylène bis-benzotriazolyl tétraméthylbutylphénol (nano), de dérivés d'acrylate de diphényle sélectionnés parmi l'octocrylène, d'oxydes métalliques sélectionnés parmi le dioxyde de titane, l'oxyde de zinc, le dioxyde de titane (nano), l'oxyde de zinc (nano), de dérivés de camphre sélectionnés parmi le méthosulfate de benzalkonium de camphre ; l'acide benzylidène camphre sulfonique ; le polyacrylamidométhyl benzylidène camphre ; le 4-méthylbenzylidène camphre ou le méthoxypropylamino cyclohexénylidène éthoxyéthylcyanoacétate, le polysilicone-15, le bis-(diéthylaminohydroxybenzoylbenzoyl) pipérazine (HAA299) et de mélanges de ces dérivés et composés.

7. Composition à utiliser selon l'une quelconque des revendications 1 à 6, dans laquelle ladite composition a un facteur de protection solaire allant de 10 à 50+, de préférence de 30 à 50.

8. Composition à utiliser selon l'une quelconque des revendications 1 à 7, dans laquelle le composé 2,6-naphtalate de diéthylhexyle est en une quantité de 0,1 à 15 % en poids, le composé benzoate de diéthylamino hydroxybenzoyl hexyle est en une quantité de 0,1 à 15 % en poids, le composé tétrasulfonate de phényl dibenzimidazole, de préférence sous forme de sel, par exemple de sel de sodium, est en une quantité de 0,1 à 15 % en poids par rapport au poids total de la composition.

9. Composition à utiliser selon l'une quelconque des revendications 1 à 8, dans laquelle ladite composition a un facteur de protection solaire allant de 15 à 30, de préférence de 15 à 20.

**10.** Composition à utiliser selon l'une quelconque des revendications 1 à 9, dans laquelle ladite composition ne comprend pas de filtres UVB.

**11.** Utilisation cosmétique non thérapeutique d'une composition pour application topique pour améliorer l'aspect esthétique de la peau affectée par une rougeur de la peau, un rinçage de la peau, dans laquelle ladite composition comprend une combinaison des composés 2,6-naphtalate de diéthylhexyle, benzoate de diéthylamino hydroxybenzoyl hexyle, tétrasulfonate de phényl dibenzimidazole et leurs sels cosmétiquement acceptables, et un véhicule cosmétiquement acceptable pour protéger le rayonnement ultraviolet, et permettant le passage du rayonnement ultraviolet ayant une longueur d'onde dans la plage de 295 à 320 nm, au moins partiellement.

**12.** Utilisation cosmétique non thérapeutique selon la revendication 11, dans laquelle ladite utilisation est destinée à protéger les rayonnements ultraviolets et à permettre le passage, au moins partiel, de rayonnements ultraviolets de longueurs d'onde dans la plage de 300 à 315 nm, de préférence de 311 à 313 nm.

FIGURE 1

FIGURE 2

FIGURE 3

FIGURE 4

FIGURE 5

FIGURE 6

**EP 4 518 849 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9968800 B2
- US 2014323950 A1
- WO 2010076731 A2

**Non-patent literature cited in the description**

- **BRIAN L. DIFFEY** ; **J. ROBSON DEL**. *J. Soc. Cosmet. Chem.*, May 1989, vol. 40, 127-133